# EUROPEAN PATENT APPLICATION

(11) **EP 4 089 164 A1**
(43) Date of publication of application: **16.11.2022**
(21) Application number: 20911865.2
(22) Date of filing: 24.11.2020
(51) Int. Cl.: C12N 5/0775, A61K 35/28, A61P 37/00

(54) **COMPOSITION FOR REINFORCING FUNCTION OF STEM CELL**

(30) Priority: 06.01.2020 KR 20200001466
(71) Applicant: SCM Lifescience Co., Ltd., Incheon 21999 (KR)
(72) Inventor: SONG, Sun Uk, Incheon 22003 (KR); KIM, Si Na, Incheon 21997 (KR); CHOI, Byeol, Incheon 21363 (KR)
(74) Representative: Altmann Stößel Dick Patentanwälte PartG mbB
(86) International application number: PCT/KR2020/016695
(87) International publication number: WO 2021/141237

(57) **Abstract**

The present invention relates to a composition, comprising: IL-1β and vitamin B6; or IL-1β, vitamin B6, and IFN-α, for promoting immunomodulatory activity and inflammation-modulating ability of stem cells, and a method for preparing stem cells having improved immunomodulatory activity and inflammation-modulating ability by using same. When treated with IL-1β and vitamin B6; or IL-1β, vitamin B6, and IFN-α according to the present invention, stem cells are induced to increase IFN-γ inhibition, inducible costimulatory ligand (ICOSL) expression, indoleamine 2,3-dioxygenase (IDO) expression, and Galectin 1 expression, and thus can find various applications in a variety of immune disease treatment fields using stem cells.

## Description

### [Technical Field]

The present invention relates to a composition for use in immune regulation and inflammation regulation ability enhancement of stem cells, the composition including Interleukin 1 beta (IL-1β) and vitamin B6; or IL-1β, vitamin B6, and interferon alpha (IFN-α) and a method for producing stem cells with improved immune regulation and inflammation regulation ability using the same.

### [Background Art]

Stem cells are undifferentiated cells and refer to cells that can divide for a long time through self-renewal and differentiate into various types of cells under a specific environment. Stem cells can be divided into embryonic stem cells and adult stem cells according to the tissue of origin. While treatment experiments using embryonic stem cells are problematic due to ethical aspects and the possibility of tumor formation, adult stem cells have the advantage that they can be easily obtained from various tissues, so research is being actively conducted to apply them to the treatment of various diseases.

So far, many compound immunosuppressants or anti-inflammatory agents have been developed and clinically most commonly used immunosuppressants include cyclosporine (Neoral, Cipol A), azathioprine (imuran), and prednisolone (a kind of steroid). The immunosuppressive agent inhibits several processes such as antigen phagocytosis by macrophage, antigen recognition by lymphocyte, cell division, division of T cells and B cells, and antibody production during the course from antigen stimulation to antibody production, causing immunosuppression. Most of them have antitumor activity because they inhibit cell division by mediating DNA disorder, DNA synthesis inhibition and the like. However, there have been problems such as hypertension and nephrotoxicity (lowered renal function), which are typical side effects, and the incidence of these side effects is high, so it is necessary to observe the progress sufficiently on use. Other side effects include tremors, seizure, hepatitis, bile retention, increased blood uric acid, decreased muscle strength, hypertrichosis, gingival hypertrophy and the like. Azathioprine, a commonly used inhibitor, may develop complications such as an inhibitory function of the bone marrow such as decreased leukocyte levels, anemia, and thrombocytopenia as well as pancreatitis, hepatitis, and bile retention and rare complications such as hair loss and fever. Prednisolone, which is one of the steroids, started to be used first among immunosuppressants, but it is a drug to be cautious because it not only accelerates arteriosclerosis but also causes hypertension, gastric ulcer, diabetes, growth inhibition, osteoporosis, cataract, and glaucoma. Thus, there is a need for a safe inhibitor or an anti-inflammatory agent.

In order to solve this problem, treatments using stem cells have recently been attempted for the treatment of inflammatory and immune diseases. In particular, mesenchymal stem cells (MSCs) are known to inhibit inflammation, induce the generation of regulatory T cells (Treg), or induce the death of immune cells involved in apoptosis. Thus, the development of various therapeutic agents using the same is being actively carried out.

However, there have been reported problems that the effect persistence or the survival rate *in vivo* are extremely low as the long-term follow-up results in various clinical trials using stem cells. Accordingly, various attempts have been made to improve stem cell therapeutics. For example, a method of inserting a specific gene into stem cells to increase the therapeutic effect, a method of pretreatment with various chemicals and peptides thatcan boost stem cell function, a method of adding stimulation conditions such as hypoxia, temperature, and light during culture are being tried, or methods of administering together with support to increase the survival rate of stem cells are being studied. Among various studies for enhancing stem cell function, a function improvement method using genetic manipulation can be effective, but problems such as the safety of the introduced gene are pointed out.

Therefore, various studies are being attempted to enhance the intrinsic function of stem cells through the treatment of various priming elements during culture rather than introducing genes into stem cells themselves to enhance their functions.

### [Disclosure]

### [Technical Problem]

Accordingly, the present inventors had studied various priming methods for enhancing the inflammatory regulation and immune regulation ability of stem cells and completed the present invention by confirming that the stem cells were treated with IL-1β and vitamin B6; or IL-1β, vitamin B6 and IFN-α to effectively enhance the function of stem cells.

Therefore, an object of the present invention is to provide a composition for enhancing the immune regulation and inflammation regulation ability of stem cells including IL-1β and vitamin B6; or IL-1β, vitamin B6 and IFN-α, a method for producing stem cells with enhanced immune regulation and inflammation regulation ability using the same, and stem cells with enhanced immune regulation and inflammation regulation ability prepared by this method.

### [Technical Solution]

In order to achieve the above object, the present invention provides a composition for enhancing immune regulation and inflammation regulation ability of stem cells including IL-1β and vitamin B6.

In addition, the present invention provides a method for enhancing immune regulation and inflammation regulation ability of stem cells, the method including step of 1) treating the stem cells with IL-1β and vitamin B6 and culturing the stem cells.

In addition, the present invention provides a method for producing the stem cells with enhanced immune regulation and inflammation regulation ability, the method including step of 1) treating the stem cells with IL-1β and vitamin B6 and culturing the stem cells.

In addition, the present invention provides a stem cell with enhanced immune regulation and inflammation regulation ability prepared by the method.

In addition, the present invention provides a pharmaceutical composition for use in preventing or treating immune diseases, the composition including stem cells with enhanced immune regulation and inflammation regulation ability.

In addition, the present invention includes a method for preventing or treating an immune disease, the method including step of treating the stem cells with enhanced immune regulation and inflammation regulation ability to the subject.

### [Advantageous Effects]

When treated with IL-1β and vitamin B6; or IL-1β, vitamin B6, and IFN-α according to the present invention, the stem cells may induce to increase IFN-γ inhibition, inducible costimulatory ligand (ICOSL) expression, indoleamine 2,3-dioxygenase (IDO) expression, and Galectin 1 expression, and thus can find various applications in a variety of immune disease treatment fields using stem cells.

### [Description of Drawings]

FIG. 1 is a view showing the results of confirming the increase in IDO expression level according to the complex treatment for each concentration of IL-1β and vitamin B6 (P value = ^{∗} < 0.1).
FIG. 2 is a view showing the results of confirming the increase in IDO expression level according to the complex treatment for each concentration of IL-1β and IFN-α (P value = ^{∗} < 0.1).
FIG. 3 is a view showing the results of confirming the IFN-γ inhibitory ability according to single or combined treatment for IL-1β, vitamin B6 and IFN-α (P value = ^{∗∗∗} < 0.001, ^{∗∗∗∗}, ####, $$$$, &&&& < 0.0001).
FIG. 4 is a view showing the result of confirming the increase in ICOSL expression level according to single or combined treatment for the IL-1β, vitamin B6 and IFN-α (P value = ^{∗} < 0.1, ^{∗∗}, ## < 0.01, &&& < 0.001).
FIG. 5 is a view showing the result of confirming the increase in Galectin 1 expression level according to single or combined treatment for IL-1β, vitamin B6 and IFN-α (P value = ^{∗} < 0.1, ## << 0.01, ^{∗∗∗∗}, ####, $$$$ < 0.0001).
FIG. 6 is a view showing the results of confirming the increase in IDO expression level according to single or combined treatment for IL-1β, vitamin B6 and IFN-α (P value = $ < 0.1, ^{∗∗} < 0.01, ^{∗∗∗}, ###, &&& < 0.001).

### [Best Mode]

Hereinafter, the present invention will be described in detail.

According to an aspect of the present invention, the present invention provides a composition for enhancing the immune regulation and inflammation regulation ability of stem cells including IL-1β and vitamin B6; or IL-1β, vitamin B6 and IFN-α.

In addition, the present invention provides a for enhancing the immune regulation and inflammation regulation ability of stem cells by IL-1β and vitamin B6; or IL-1β, vitamin B6 and IFN-α.

IL-1β and vitamin B6; or IL-1β, vitamin B6 and IFN-α of the present invention may enhance the immune regulation and inflammation regulation ability of stem cells to induce function-enhancing stem cells.

The combination of IL-1β and vitamin B6; or the combination of IL-1β, vitamin B6 and IFN-α exhibits a synergistic effect compared to treatment of stem cells with a single component thereof and may effectively induce enhancement of the function of the desired stem cell even at a low treatment concentration.

Specifically, in order to induce function-enhancing of stem cells, the stem cells may be treated with a combination of IL-1β and vitamin B6 and then cultured. IL-1β and vitamin B6 may be included in the composition in a ratio of 1 : 1 to 1 : 6,000 (w/v). IL-1β and vitamin B6 may be preferably included in a ratio of 1 : 60 to 1 : 6,000 (w/v), more preferably in a ratio of 1 : 1,000 to 6,000 (w/v). In one embodiment of the present invention, 20 ng/ml of IL-1β and 25 to 100 µg/ml of vitamin B6 were complexly treated to confirm the synergistic effect of enhancing the immune regulation and inflammation regulation function of stem cells.

Further, in order to more significantly induce function-enhancing of stem cells in the present invention, the stem cells may be treated with a combination of IL-1β, vitamin B6 and IFN-α and then cultured. IL-1β, IFN-α and vitamin B6 may be included in the composition in a ratio of 1 : 0.01 to 5 : 1 to 1 : 6,000 (w/v). IL-1β, IFN-α and vitamin B6 may be more preferably included in a ratio of 1 : 0.05 to 2 : 1,000 to 6,000 (w/v). In one embodiment of the present invention, 20 ng/ml of IL-1β, 20 ng/ml of IFN-α, and 50 µg/ml of vitamin B6 were selected as concentrations for complex treatment, and stem cells were treated with IL-1β, vitamin B6 and IFN-α to confirm the enhancement of immune regulation and inflammation regulation function.

It was confirmed that in particular, when stem cells were treated with a combination of IL-1β, IFN-α and vitamin B6, the immune regulation and inflammation regulation functions may be very remarkably enhanced such as IFN-γ inhibition enhancement, inducible costimulatory ligand (ICOSL) expression increase, indoleamine 2,3-dioxygenase (IDO) expression increase, and Galectin 1 expression increase compared with the results of treatment with individual substances on stem cells. Accordingly, it was confirmed that this combination is the optimal combination for function-enhancing of stem cells.

In addition, the combination of IL-1β, IFN-α and vitamin B6 may further enhance the expression of TSG6 or Galectin 9, which are markers of stem cell efficacy.

In the present invention, stem cells whose function is enhanced by treatment with IL-1β and vitamin B6; or IL-1β, vitamin B6 and IFN-α refer to cells having the ability to differentiate into two or more cells while having self-renewal ability and may be classified into totipotent stem cells, pluripotent stem cells and multipotent stem cells. The stem cells may be appropriately selected without limitation depending on the purpose and may be derived from adult cells such as all known tissues and cells derived from mammals including humans, preferably humans. For example, it may be adipose, bone marrow, placental (or placental tissue cells), or umbilical cord blood-derived mesenchymal stem cells.

The function of stem cells may be enhanced by treating and culturing the stem cells with IL-1β and vitamin B6; or IL-1β, vitamin B6 and IFN-α according to the present invention. In the present invention, enhancing the function of stem cells means boosting the ability to regulate inflammation and regulate immune that stem cells have inherently, and IL-1β and vitamin B6; or IL-1β, vitamin B6 and IFN-α may be utilized as priming agents for boosting stem cells. More specifically, the function-enhancing is inflammation regulation and immunomodulatory enhancement, which may mean at least one kind of immune regulation and inflammation regulation ability enhancement of stem cells selected from the group consisting of IFN-γ inhibition enhancement, inducible costimulatory ligand (ICOSL) expression increase, indoleamine 2,3-dioxygenase (IDO) expression increase, and Galectin 1 expression increase.

The present invention provides a method for enhancing immune regulation and inflammation regulation ability of stem cells, the method including steps of 1) treating the stem cells with IL-1β and vitamin B6 and culturing the stem cells.

In addition, the present invention includes a method for preparing stem cells with enhanced immune regulation and inflammation regulation ability, the method including steps of 1) treating the stem cells with IL-1β and vitamin B6 and culturing the stem cells.

In order to further enhance immune regulation and inflammation regulation ability, the present invention may be characterized in that IL-1β and vitamin B6 are additionally treated with IFN-α and may be treated simultaneously or sequentially.

The method may be carried out *in vitro* or *ex vivo,* and the culture may be carried out in a medium containing the components necessary for stem cell culture well known in the art or medium that additionally includes a component capable of promoting the proliferation of stem cells. The stem cells of the present invention may be grown in a normal medium by additionally adding and mixing a substance for a special purpose, which includes a material containing nutrients required by the cells to be cultured, i.e., to-be-cultured cells in order to culture the stem cells of the present invention. The medium is also referred to as an incubator or culture medium, and is a concept including all of natural medium, synthetic medium, or selective medium. For example, in the present invention, Dulbecco's modified Eagle medium (DMEM) or alpha-MEM may be used, but is not limited thereto. Other culture conditions, such as temperature and culture time, may follow the normal culture conditions of mesenchymal stem cells.

In the method of the present invention, content overlapping with the composition for enhancing immune regulation and inflammation regulation is excluded in order to avoid the complexity of the description.

In addition, the present invention provides stem cells with enhanced immune regulation and inflammation regulation ability prepared by the above production method.

The stem cells are characterized by having enhanced function by IL-1β and vitamin B6; or IL-1β, vitamin B6 and IFN-α treatment which has enhanced immune regulation and inflammation regulation ability compared to the untreated stem cells.

More specifically, the stem cells of the present invention may be characterized by enhancing at least one kind of immune regulation and inflammation regulation ability enhancement of stem cells selected from the group consisting of IFN-γ inhibition enhancement, inducible costimulatory ligand (ICOSL) expression increase, indoleamine 2,3-dioxygenase (IDO) expression increase, and Galectin 1 expression increase compared to the untreated stem cells.

Since the stem cells of the present invention have enhanced immune regulation and inflammatory regulation ability, they may be used for the treatment of, various immune diseases and inflammatory diseases related to inflammation-related mechanisms and immune-modulating mechanisms, and in particular may be usefully used for the prevention or treatment of immune diseases.

Accordingly, the present invention provides a pharmaceutical composition for use in preventing or treating immune diseases, including the stem cells.

In addition, the present invention provides a method for preventing or treating immune diseases including step of administering the stem cells to a subject, preferably a subject in need thereof.

In the present invention, the immune disease may be a disease mediated by IDO or ICOSL with enhanced expression ability in the stem cells of the present invention and could be a disease caused by not normally expressing the IDO and ICOSL or not secreting their proteins on immune or inflammation-related mechanisms.

More specifically, the immune diseases may include at least one kind selected from the group consisting of Crohn's disease, erythema, atopy, rheumatoid arthritis, Hashimoto's thyroiditis, pernicious anemia, Addison's disease, type 1 diabetes, lupus, chronic fatigue syndrome, fibromyalgia, hypothyroidism, hyperthyroidism, scleroderma, Behcet's disease, inflammatory bowel disease, multiple sclerosis, myasthenia gravis, Meniere's syndrome, Guillain-Barre syndrome, Sjogren's syndrome, vitiligo, endometriosis, psoriasis, systemic scleroderma, asthma and ulcerative colitis.

Since stem cells, which are active ingredients of the pharmaceutical composition of the present invention are stem cells with enhanced immune regulation and inflammation regulation ability by treatment with IL-1β and vitamin B6; or IL-1β, vitamin B6 and IFN-α, it may exhibit a significantly superior effect compared to a therapeutic agent for immune disease containing untreated stem cells as an active ingredient.

The pharmaceutical composition of the present invention may further include suitable carriers, excipients and diluents commonly used in the preparation of pharmaceutical compositions. In addition, additives for solid or liquid formulations may be used in the preparation of the pharmaceutical composition. The additive for formulation may be either organic or inorganic.

Examples of the excipient may include lactose, sucrose, white sugar, glucose, corn starch, starch, talc, sorbit, crystalline cellulose, dextrin, kaolin, calcium carbonate, silicon dioxide, and the like. Examples of the binder may include polyvinyl alcohol, polyvinyl ether, ethyl cellulose, methyl cellulose, gum arabic, tragacanth, gelatin, shellac, hydroxypropyl cellulose, hydroxypropyl methyl cellulose, calcium citrate, dextrin, pectin, and the like. Examples of the lubricant may include magnesium stearate, talc, polyethylene glycol, silica, hydrogenated vegetable oil, and the like. As a coloring agent, if it is permitted to be added to pharmaceuticals normally, all may be used. These tablets and granules may be coated with sugar coating, gelatin coating, or other appropriate coatings according to need. In addition, preservatives, antioxidants, etc. may be added as necessary.

The pharmaceutical composition of the present invention may be prepared in any formulation conventionally prepared in the art, and the form of the formulation is not particularly limited but may preferably be an external application. The external application of the present invention may include the form of a conventional external preparation such as a sheet, a liquid coating, a spray, a lotion, a cream, a patch, a powder, a penetrating pad, a gel, a pasta, a liniment, an ointment, an aerosol, a powder, a suspension, a transdermal absorbent. These formulations are described in Remington's Pharmaceutical Science, a commonly known recipe for all pharmaceutical chemistry.

The pharmaceutically effective amount of the present invention may vary depending on the patient's wound type, application site, number of treatments, treatment time, dosage form, patient's condition, type of adjuvant, and the like. The amount is not particularly limited but may be 0.00001 to 10,000 µg when an effective daily amount of the pharmaceutical composition of the present invention is applied to a patient normally. The daily dose may be administered once a day or divided into two to three times a day at appropriate intervals or may be administered intermittently at intervals of several days.

However, the amount of the pharmaceutical composition of the present invention is determined in light of several relevant factors such as the route of administration, the patient's age, gender, weight, the severity of the patient, the type of wound, the application site, the number of treatments, the treatment time, the dosage form, the patient's condition, and the type of adjuvant, the effective amount is not to be construed as limiting the scope of the invention in any respect.

In addition, the present invention provides a method for inhibiting an immune response or an inflammatory response of a subject, including administering the composition to the subject.

In the present invention, the term "subject" refers to mammals including cattle, dogs, pigs, chickens, sheep, horses, and humans, but is not limited thereto.

Hereinafter, the present invention will be described in more detail through examples. It will be apparent to those of ordinary skill in the art that these examples are only for illustrating the present invention, and the scope of the present invention is not to be construed as being limited by these examples.

### [Modes of the Invention]

### Experimental Example 1. Selection of experimental group of stem cell function-enhancing and stem cell culture

At 37°C and 5% CO₂ incubator, mesenchymal stem cells in storage state (stored in LN2 tank) were thawed and cultured. At this time, they were cultured in a medium (DMEM, alpha-MEM) containing 10% FBS or 4% hPL. Cells were cultured until confluence reached about 80%. After seeding the cultured mesenchymal stem cells in a 100 mm dish, a candidate material for enhancing the mesenchymal stem cell function was selected.

IL-1β, IFN-α and vitamin B6 were selected as candidate substances, and concentrations for function-enhancing were set. When IL-1β was treated in MSC, it was confirmed that the lowest effective concentration capable of increasing the expression of TSG6 and IDO was 20 ng/ml, and this was set as the standard.

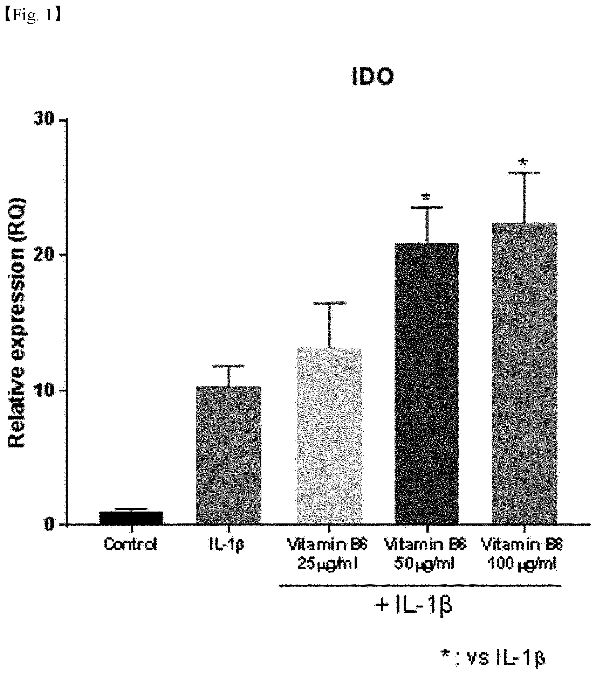

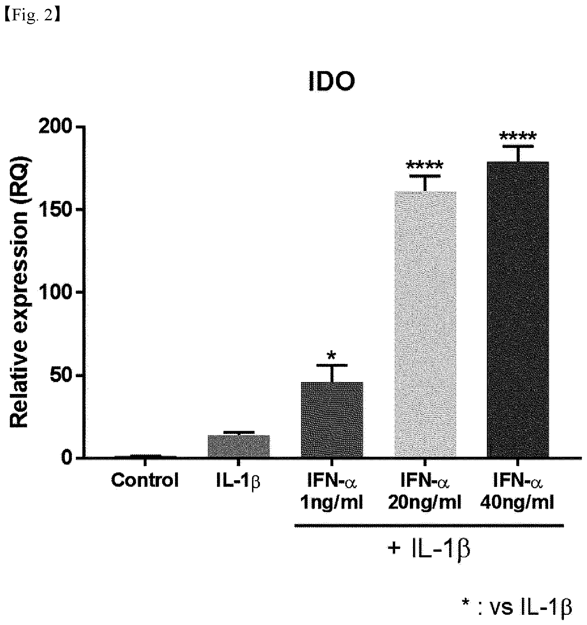

The selected 20 ng/ml of IL-1β and vitamin B6 and IFN-α at different concentrations were combined, and the effect was confirmed by the change in indoleamine 2,3-dioxygenase (IDO) expression. IDO is known as an immunomodulatory factor that suppresses the proliferation of immune cells such as T cells by changing tryptophan, which is essential for T cell proliferation, into kynurenine. After culturing stem cells, total RNA was isolated using TRIzol (Invitrogen), and cDNA was synthesized from total RNA using PrimeScript^{™} reagent Kit with gDNA Eraser (TaKaRa), followed by qRT-PCR. Through this, the expression change of IDO was confirmed, and the results are shown in FIGS. 1 and 2.

As shown in FIG. 1, as a result of treatment with 20 ng/m IL-1β by changing 25 to 100 µg/ml vitamin B6, it was confirmed that the expression of IDO increased compared to treatment with IL-1β alone in all treated experimental groups. In particular, in the 50 µg/ml to 100 µg/ml treatment group, the IDO expression increased by about 2 times or more compared to the treatment with IL-1β alone, confirming the excellent function-enhancing effect.

As shown in FIG. 2, it was confirmed that IDO expression even in the experimental group combinedly treated with IFN-α at 1 to 40 ng/ml was significantly increased compared to treatment with IL-1β alone.

Based on the above results, 5 µg/ml of vitamin B6 and 20 ng/ml of IFN-α were selected as functional enhancing substances that may be treated in combination with IL-1β. By combining these, the function-enhancing effect of mesenchymal stem cells was confirmed in the following experiment.

The selected candidate substances and their concentration combinations are shown in Table 1, and they were treated to mesenchymal stem cells for 24 hours, and the function-enhancing effect was confirmed.

**[Table 1]**

| Experimental Group | |
|---|---|
| 1 | IL-1β (20 ng/ml) |
| 2 | IFN-α (20 ng/ml) |
| 3 | Vitamin B6 (50 µg/ml) |
| 4 | IL-1β (20 ng/ml) + IFN-α (20 ng/ml) |
| 5 | IL-1β (20 ng/ml) + Vitamin B6 (50 µg/ml) |
| 6 | IFN-α (20 ng/ml) + Vitamin B6 (50 µg/ml) |
| 7 | IL-1β (20 ng/ml) + IFN-α (20 ng/ml) + Vitamin B6 (50 µg/ml) |

### Example 1. Confirmation of function-enhancement of mesenchymal stem cells according to the treatment with the experimental group

### 1.1 Confirmation of IFN-γ secretion according to the treatment with the experimental group

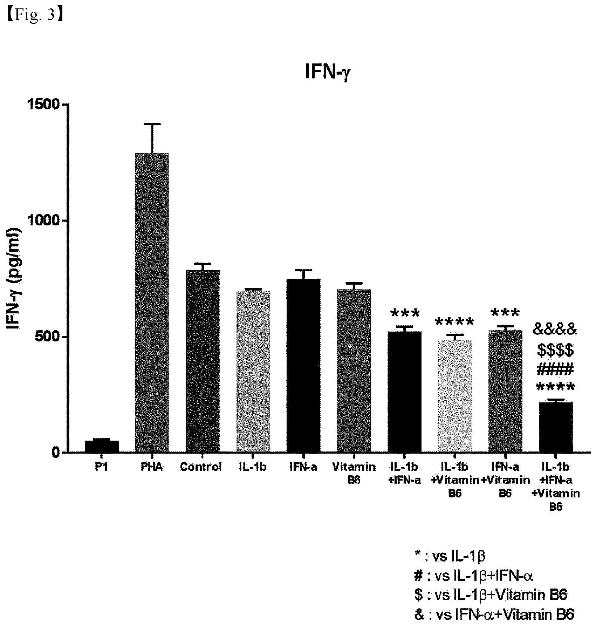

In order to confirm whether the activity of mesenchymal stem cells was changed according to the treatment with the experimental group as shown in Table 1 above, after activating T-cells in stimulation conditions of phytohemagglutinin (PHA), which are immunomodulatory active substances, mesenchymal stem cells were co-cultured for 3 days. Thereafter, the co-cultured supernatant was obtained and the secretion of IFN-γ, a pro-inflammatory cytokine, was confirmed through an IFN-γ kit (BD Biosciences) to confirm whether the mesenchymal stem cells had a change in immune regulation ability. As a control group, mesenchymal stem cells that were not treated with the test substance were used. The secretion of IFN-γ according to the treatment of the experimental group is shown in FIG. 3.

As shown in FIG. 3, IFN-γ secretion was increased by PHA stimulation, and this increase in IFN-γ was decreased by treatment of mesenchymal stem cells. On the other hand, it was confirmed that in the case of the IL-1β, IFN-α, or vitamin B6 single-treated experimental group, the change in IFN-γ secretion was not significant compared to the untreated control group. However, it was confirmed that the IL-1β + IFN-α, IL-1β + vitamin B6, and IFN-α+vitamin B6 treatment group could significantly inhibit the secretion of IFN-γ compared to the single treatment group. In addition, it was confirmed that a very significant IFN-γ secretion inhibitory effect was shown in the experimental group treated with all of IL-1β, IFN-α and vitamin B6, and it was confirmed that it exhibited secretion inhibitory ability, which was about twice or more compared to the experimental group combining the two experimental substances.

### 1.2. Comparison of expression of inflammation and immunomodulatory factors according to the treatment of the experimental group

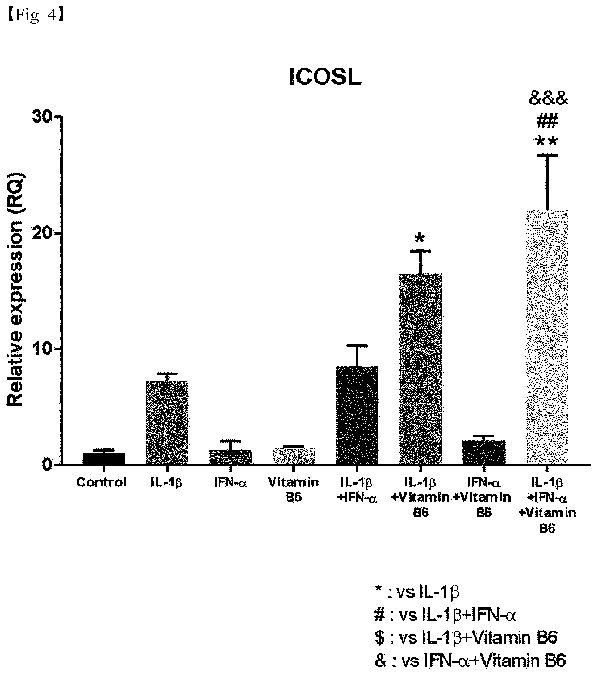

Inducible costimulatory ligand (ICOSL) is known to inhibit activated T cells by inducing regulatory T cells. Since the function-enhancing effect of the mesenchymal stem cells according to the treatment with the experimental group was confirmed in Example 1.1, an experiment was performed to additionally confirm whether the treatment with the experimental group induces inflammation and immune regulation ability of the mesenchymal stem cells. After culturing stem cells as in Experimental Example 1, total RNA was isolated using TRIzol (Invitrogen), and then cDNA was synthesized from total RNA using PrimeScript^{™} reagent Kit with gDNA Eraser (TaKaRa) and qRT-PCR was performed. Through this, the expression change of ICOSL, which is a representative factor of inflammation and immune regulation, was confirmed, and the results are shown in FIG. 4.

As shown in FIG. 4, the expression of ICOSL increased in the group treated with IL-1β single substance but did not show a distinct increase in the group treated with IFN-α or vitamin B6 alone. However, an increase in ICOSL expression was induced in the experimental group treated with combination thereof. In particular, it was confirmed that an ICOSL expression was very significantly increased in an experimental group treated with a combination of IL-1β and vitamin B6, and experimental group treated with a combination of all of IL-1β, IFN-α, and vitamin B6 compared to the group treated with IL-1β alone. This result is also consistent with the results of suppression of IFN-γ secretion confirmed in Example 1.1. These results indicate that it is possible to effectively enhance the immune and inflammatory regulation functions of mesenchymal stem cells when treating mesenchymal stem cells with a combination of IL-1β and vitamin B6 or IL-1β, IFN-α and vitamin B6.

### 1.3. Confirmation of change in Galectin 1 expression according to the treatment with the experimental group

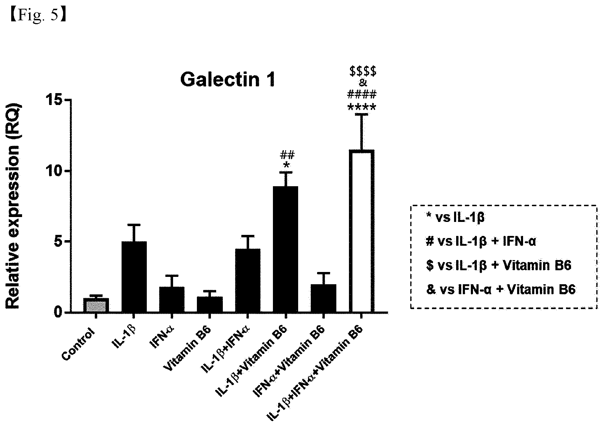

Galectin 1 is known as a stem cell potency marker closely related to the high efficacy of stem cells, and it is known that the more the expression increases, the stronger the stem cell function and efficacy. Therefore, in order to further confirm whether the treatment with the experimental group induces the inflammation and immune regulation ability of the mesenchymal stem cells, an experiment was performed to confirm the expression of Galectin 1. After culturing stem cells as in Experimental Example 1, total RNA was isolated using TRIzol (Invitrogen), and then cDNA was synthesized from total RNA using PrimeScript^{™} reagent Kit with gDNA Eraser (TaKaRa) and qRT-PCR was performed. Using this, a change in the expression of Galectin 1, an indicator of stem cell efficacy, was confirmed, and the result is shown in FIG. 5.

As shown in FIG. 5, the expression of Galectin 1 was increased in the group treated with IL-1β alone but did not show a distinct increase in the group treated with vitamin B6 alone. However, a significant increase in Galectin 1 expression was induced in the experimental group treated with a combination thereof. In particular, it was confirmed that a Galectin 1 expression was significantly increased in an experimental group treated with a combination of IL-1β and vitamin B6, and an experimental group treated with a combination of all of IL-1β, IFN-α, and vitamin B6 compared to the group treated with IL-1β alone. This is a result consistent with the IFN-γ secretion inhibition result confirmed in Example 1.1 and the ICOSL result of Example 1.2. These results indicate that it is possible to effectively enhance the immune and inflammatory regulation functions of mesenchymal stem cells when treating mesenchymal stem cells with a combination of IL-1β and vitamin B6 or IL-1β, IFN-α and vitamin B6.

### 1.4. Confirmation of change in IDO expression according to the treatment with the experimental group

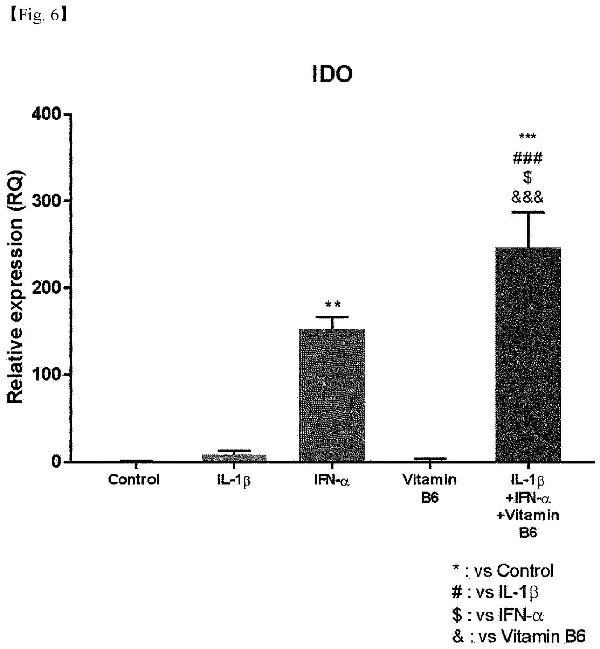

Since it was confirmed through Examples 1.2 and 1.3 that a combination of IL-1β and vitamin B6 or a combination of IL-1β, IFN-α and vitamin B6 may enhance the function of mesenchymal stem cells for regulating immunity and inflammation, additional experiments were performed on the combination of IL-1β, IFN-α and vitamin B6 showing the best effect. The combination of IL-1β, IFN-α and vitamin B6 was treated on mesenchymal stem cells, and the effect of inducing changes in IDO expression was confirmed by the same experimental method as in Example 1.2, and the results are shown in FIG. 6.

As shown in FIG. 6, IDO did not show a distinct expression change by treatment with IL-1β alone and vitamin B6 alone. However, IDO expression increased up to about 135 times or more in the experimental group treated with the combination of IL-1β, IFN-α and vitamin B6 in mesenchymal stem cells, compared to the mesenchymal stem cells treated with individual test substances.

Combining the experimental results as described above, it may be seen that when mesenchymal stem cells were treated with the combination of IL-1β and vitamin B6 or the combination of IL-1β, IFN-α and vitamin B6, the ability of mesenchymal stem cells to regulate immunity and inflammation was improved. Therefore, the combination of IL-1β and vitamin B6 or the combination of IL-1β, IFN-α and vitamin B6 may be utilized as a priming factor for enhancing the inflammatory regulation and immune regulation function of mesenchymal stem cells.

Above, specific parts of the present invention have been described in detail. It is clear for those of ordinary skill in the art that these specific descriptions are only preferred embodiments, and the scope of the present invention is not limited thereby. Accordingly, it is intended that the substantial scope of the present invention be defined by the appended claims and their equivalents.

## Claims

1. A composition for use in immune regulation and inflammation regulation ability enhancement of stem cells, the composition comprising IL-1β and vitamin B6.

2. The composition of claim 1, further comprising IFN-α.

3. The composition of claim 1, wherein the IL-1β and vitamin B6 are included in a ratio of 1 : 1 to 1 : 6,000 (w/v).

4. The composition of claim 2, wherein the IL-1β, IFN-α and vitamin B6 are included in a ratio of 1 : 0.01 to 5 : 1 to 6,000 (w/v).

5. The composition of any one of claims 1 to 4, wherein the stem cells are mesenchymal stem cells derived from fat, bone marrow, placenta, or umbilical cord blood.

6. The composition of claim 1 or 2, wherein the IL-1β and vitamin B6; or IL-1β, IFN-α and vitamin B6 induce at least one kind of immune regulation and inflammation regulation ability enhancement of stem cells selected from the group consisting of IFN-γ inhibition enhancement, inducible costimulatory ligand (ICOSL) expression increase, indoleamine 2,3-dioxygenase (IDO) expression increase, and Galectin 1 expression increase.

7. A method for enhancing immune regulation and inflammation regulation ability of stem cells, the method comprising step of:
1) treating the stem cells with IL-1β and vitamin B6 and then culturing the stem cells.

8. The method of claim 7, wherein IFN-α is co-treated in step 1).

9. The method of claim 7, wherein the immune regulation and inflammation regulation ability enhancement of stem cells include at least one kind selected from the group consisting of IFN-γ inhibition enhancement, inducible costimulatory ligand (ICOSL) expression increase, indoleamine 2,3-dioxygenase (IDO) expression increase, and Galectin 1 expression increase.

10. A method for producing stem cells with enhanced immune regulation and inflammation regulation ability, the method comprising step of:
1) treating the stem cells with IL-1β and vitamin B6 and then culturing the stem cells.

11. The method of claim 10, wherein IFN-α is co-treated in step 1).

12. A stem cell with enhanced immune regulation and inflammation regulation ability prepared by the method of claim 10 or 11.

13. A pharmaceutical composition for use in preventing or treating immune diseases, comprising the stem cell of claim 12.

14. The pharmaceutical composition of claim 13, wherein the immune disease includes at least one kind selected from the group consisting of Crohn's disease, erythema, atopy, rheumatoid arthritis, Hashimoto's thyroiditis, pernicious anemia, Addison's disease, type 1 diabetes, lupus, chronic fatigue syndrome, fibromyalgia, hypothyroidism, hyperthyroidism, scleroderma, Behcet's disease, inflammatory bowel disease, multiple sclerosis, myasthenia gravis, Meniere's syndrome, Guillain-Barre syndrome, Sjogren's syndrome, vitiligo, endometriosis, psoriasis, systemic scleroderma, asthma and ulcerative colitis.

15. A method of preventing or treating an immune disease, the method comprising a step of treating the stem cell of claim 12 to a subject.
